# EUROPEAN PATENT APPLICATION

(11) **EP 1 990 011 A1**
(43) Date of publication of application: **12.11.2008**
(21) Application number: 08155420.6
(22) Date of filing: 30.04.2008
(51) Int. Cl.: A61B 8/00, G06F 19/00, A61B 5/00

(54) **Ultrasonic diagnostic apparatus main body unit, operation unit and ultrasonic diagnostic apparatus**

(30) Priority: 08.05.2007 JP 2007122916
(71) Applicant: GE Medical Systems Global Technology Company, LLC, Waukesha, Wisconsin 53188-1696 (US)
(72) Inventor: Koide, Tetsuo GE Yokogawa Medical Systems, Ltd., Hino-shi Tokyo 191-8503 (JP); Morita, Dai GE Yokogawa Medical Systems, Ltd., Hino-shi Tokyo 191-8503 (JP); Amemiya, Shinichi GE Yokogawa Medical Systems, Ltd., Hino-shi Tokyo 191-8503 (JP); Yanagihara, Koji GE Yokogawa Medical Systems, Ltd., Hino-shi Tokyo 191-8503 (JP)
(74) Representative: Bedford, Grant Richard

(57) **Abstract**

An ultrasonic diagnostic apparatus main body unit (20) includes: a main body wireless communication device (27) for wireless communicating with separate operation units (40); and a main body control device (26) which accepts operation instructions given from the plural operation units (40) and activates the function of an ultrasonic diagnostic apparatus (101) in accordance with the operation instructions.

## Description

The present invention relates generally to an ultrasonic diagnostic apparatus main body unit, an operation unit and an ultrasonic diagnostic apparatus, and more specifically to an ultrasonic diagnostic apparatus main body unit capable of operating one ultrasonic diagnostic apparatus main body unit by a plurality of operation units, an operation unit and an ultrasonic diagnostic apparatus, and to an ultrasonic diagnostic apparatus main body unit capable of operating a plurality of ultrasonic diagnostic apparatus main body units by one operation unit, an operation unit and an ultrasonic diagnostic apparatus.

There has heretofore been known an ultrasonic diagnostic apparatus capable of operating an ultrasonic diagnostic apparatus main body unit by wireless communications from an operation unit (refer to, for example, patent documents 1 and 2).
[Patent Document 1] Japanese Unexamined Patent Publication No. 2002-085405
[Patent Document 2] Japanese Unexamined Patent Publication No. 2003-153903

When a doctor provides living lessons to a sonographer (person who performs the work of scanning a subject with ultrasound), for example, it is preferable that the sonographer located in the vicinity of a patient and the doctor located slightly away from the sonographer are able to operate an ultrasonic diagnostic main body unit substantially simultaneously.

When, for example, one doctor performs ultrasonic diagnoses on a plurality of patients substantially simultaneously, it is preferable that ultrasonic diagnostic apparatus main body units and sonographers are disposed every patient and one doctor is able to operate the plural ultrasonic diagnostic apparatus main body units substantially simultaneously.

However, the conventional ultrasonic diagnostic apparatus was accompanied by a problem that it could not cope with such a case as referred to above because the operation unit and the ultrasonic diagnostic apparatus main body unit were in a one-to-one correspondence.

It is desirable that the problem described previously is addressed.

In a first aspect, the invention provides an ultrasonic diagnostic apparatus main body unit comprising a main body wireless communication device for wireless communicating with separate operation units, and a main body control device which accepts operation instructions given from the plural operation units via the main body wireless communication device and activates the function of an ultrasonic diagnostic apparatus in accordance with the operation instructions.

The ultrasonic diagnostic apparatus main body unit according to the first aspect accepts operation instructions issued from a plurality of operation units via a main body wireless communication device and activates the function of an ultrasonic diagnostic apparatus in accordance with the operation instructions. Therefore, for example, a sonographer located in the vicinity of a patient and a doctor located slightly away from the sonographer is able to operate the ultrasonic diagnostic apparatus main body unit substantially simultaneously. The invention is useful for allowing the doctor to make living lessons to the sonographer.

Incidentally, the wireless communications are, for example, communications by radio waves or communications by infrared light.

In a second aspect, the invention provides an ultrasonic diagnostic apparatus main body unit wherein in the ultrasonic diagnostic apparatus main body unit according to the first aspect, an operation unit limiting device for limiting each operation unit that accepts the operation instructions is provided.

In the ultrasonic diagnostic apparatus main body unit according to the second aspect, the occurrence of misoperations can be suppressed because each operation unit that accepts operation instructions can be limited.

In a third aspect, the invention provides an ultrasonic diagnostic apparatus main body unit wherein in the ultrasonic diagnostic apparatus main body unit according to the second aspect, an operation unit display device for displaying each operation unit that accepts the operation instructions is provided.

In the ultrasonic diagnostic apparatus main body unit according to the third aspect, it is possible to visually identify which operation unit is active, since each operation unit that accepts the operation instructions is displayed.

In a fourth aspect, the invention provides an ultrasonic diagnostic apparatus main body unit wherein in the ultrasonic diagnostic apparatus main body unit according to the second or third aspect, an operation unit limit releasing device for releasing the limitation of each operation unit that accepts the operation instructions is provided.

In the ultrasonic diagnostic apparatus main body unit according to the fourth aspect, it can be operated by any operation unit by releasing the limitation of each operation unit where it cannot be operated by all operation units.

In a fifth aspect, the invention provides an ultrasonic diagnostic apparatus comprising the ultrasonic diagnostic apparatus main body unit according to any of the first through fourth aspects, and a plurality of operation units separate from the main body unit, wherein each of the operation units includes an operation wireless communication device for wireless communicating with the main body unit, an operation device for inputting operation instructions to the main body unit by an operator, and an operation controller which sends out the operation instructions to the main body unit via the operation wireless communication device.

In the ultrasonic diagnostic apparatus according to the fifth aspect, the function of the ultrasonic diagnostic apparatus can be activated in accordance with operation instructions issued from a plurality of operation units. Therefore, for example, a sonographer located in the vicinity of a patient and a doctor located slightly away from the sonographer is able to operate the ultrasonic diagnostic apparatus main body unit substantially simultaneously. The invention is useful for allowing the doctor to make living lessons to the sonographer.

In a sixth aspect, the invention provides an ultrasonic diagnostic apparatus main body unit comprising a main body wireless communication device for wireless communicating with separate operation units, and a main body control device which accepts operation instructions issued from the plural operation units via the main body wireless communication device, activates the function of an ultrasonic diagnostic apparatus in accordance with the operation instructions, and sends display data to the operation units.

The ultrasonic diagnostic apparatus main body unit according to the sixth aspect accepts operation instructions issued from a plurality of operation units via a main body wireless communication device and activates the function of an ultrasonic diagnostic apparatus in accordance with the operation instructions. Therefore, for example, a sonographer located in the vicinity of a patient and a doctor located slightly away from the sonographer is able to operate the ultrasonic diagnostic apparatus main body unit substantially simultaneously. The invention is useful for allowing the doctor to make or provide living lessons to the sonographer. Since display data is sent out to each operation unit, the contents of operations and the results of operations can be displayed on the operation unit side.

In a seventh aspect, the invention provides an ultrasonic diagnostic apparatus main body unit wherein in the ultrasonic diagnostic apparatus main body unit according to the sixth aspect, an operation unit limiting device for limiting each operation unit that accepts the operation instructions is provided.

In the ultrasonic diagnostic apparatus main body unit according to the seventh aspect, the occurrence of misoperations can be suppressed because each operation unit that accepts operation instructions can be limited.

In an eighth aspect, the invention provides an ultrasonic diagnostic apparatus main body unit wherein in the ultrasonic diagnostic apparatus main body unit according to the seventh aspect, an operation unit display device for displaying each operation unit that accepts the operation instructions is provided.

In the ultrasonic diagnostic apparatus main body unit according to the eighth aspect, it is possible to visually identify which operation unit is active, since each operation unit that accepts the operation instructions is displayed.

In a ninth aspect, the invention provides an ultrasonic diagnostic apparatus main body unit wherein in the ultrasonic diagnostic apparatus main body unit according to the seventh or eighth aspect, an operation unit limit releasing device for releasing the limitation of each operation unit that accepts the operation instructions is provided.

In the ultrasonic diagnostic apparatus main body unit according to the ninth aspect, it can be operated by any operation unit by releasing the limitation of each operation unit where it cannot be operated by all operation units.

In a tenth aspect, the invention provides an ultrasonic diagnostic apparatus comprising the ultrasonic diagnostic apparatus main body unit according to any of the sixth through ninth aspects, and a plurality of operation units separate from the main body unit, wherein each of the operation units includes an operation wireless communication device for wireless communicating with the main body unit, an operation device for inputting operation instructions to the main body unit by an operator, an operation controller which sends out the operation instructions to the main body unit via the operation wireless communication device and accepts display data sent from the main body unit, and a display device which performs a display based on the display data.

In the ultrasonic diagnostic apparatus according to the tenth aspect, the function of the ultrasonic diagnostic apparatus can be activated in accordance with operation instructions issued from a plurality of operation units. Therefore, for example, a sonographer located in the vicinity of a patient and a doctor located slightly away from the sonographer is able to operate the ultrasonic diagnostic apparatus substantially simultaneously. The invention is useful for allowing the doctor to make living lessons to the sonographer. The contents of operations and the results of operations can be displayed on the operation unit side.

In an eleventh aspect, the invention provides an operation unit comprising an operation wireless communication device for wireless communicating with a plurality of separate ultrasonic diagnostic apparatus main body units, an operation device for designating one of the main body units by an operator and inputting operation instructions to the corresponding main body unit by the operator, and an operation controller which sends the operation instructions to the designated main body unit via the operation wireless communication device.

In the operation unit according to the eleventh aspect, the function of a designated ultrasonic diagnostic apparatus main body unit of a plurality of ultrasonic diagnostic apparatus main body units can be activated in accordance with operation instructions given from each operation unit. Therefore, for example, ultrasonic diagnostic apparatus main body units and sonographers are disposed every patient, and one doctor operates the plural ultrasonic diagnostic apparatus main body units substantially simultaneously, whereby one doctor is able to perform ultrasonic diagnoses on a plurality of patients substantially simultaneously.

In a twelfth aspect, the invention provides an operation unit wherein in the operation unit according to the eleventh aspect, a designated device display device for displaying the main body unit placed during designation, as a destination for sending of the operation instructions is provided.

In the operation unit according to the twelfth aspect, it is possible to visually identify for which ultrasonic diagnostic apparatus main body unit the operation unit is active, since the ultrasonic diagnostic apparatus main body unit placed during designation is displayed.

In a thirteenth aspect, the invention provides an ultrasonic diagnostic apparatus comprising the operation unit according to the eleventh or twelfth aspect, and a plurality of ultrasonic diagnostic apparatus main body units separate from the operation unit, wherein each of the ultrasonic diagnostic apparatus main body units includes a main body wireless communication device for wireless communicating with the operation unit, and a main body control device for accepting operation instructions issued from the operation unit via the main body wireless communication device and activating the function of the ultrasonic diagnostic apparatus in accordance with the operation instructions.

In the ultrasonic diagnostic apparatus according to the thirteenth aspect, the function of a designated ultrasonic diagnostic apparatus main body unit of a plurality of ultrasonic diagnostic apparatus main body units can be activated in accordance with operation instructions given from each operation unit. Therefore, for example, ultrasonic diagnostic apparatus main body units and sonographers are disposed every patient, and one doctor operates the plural ultrasonic diagnostic apparatus main body units substantially simultaneously, whereby one doctor is able to perform ultrasonic diagnoses on a plurality of patients substantially simultaneously.

In a fourteenth aspect, the invention provides an operation unit comprising an operation wireless communication device for wireless communicating with a plurality of separate ultrasonic diagnostic apparatus main body units, an operation device for designating one of the main body units by an operator and inputting operation instructions to the corresponding main body unit by the operator, an operation controller which sends the operation instructions to the designated main body unit via the operation wireless communication device and accepts display data sent from the designated main body unit, and a display device which performs a display based on the display data.

In the operation unit according to the fourteenth aspect, the function of a designated ultrasonic diagnostic apparatus main body unit of a plurality of ultrasonic diagnostic apparatus main body units can be activated in accordance with operation instructions given from each operation unit. Therefore, for example, ultrasonic diagnostic apparatus main body units and sonographers are disposed every patient, and one doctor operates the plural ultrasonic diagnostic apparatus main body units substantially simultaneously, whereby one doctor is able to perform ultrasonic diagnoses on a plurality of patients substantially simultaneously. Further, since display data is sent out to each operation unit, the contents of operations and the results of operations can be displayed on the operation unit side.

In a fifteenth aspect, the invention provides an operation unit wherein in the operation unit according to the fourteenth aspect, a designated device display device for displaying the main body unit placed during designation, as a destination for sending of the operation instructions is provided.

In the operation unit according to the fifteenth aspect, it is possible to visually identify for which ultrasonic diagnostic apparatus main body unit the operation unit is active, since the ultrasonic diagnostic apparatus main body unit placed during designation is displayed.

In a sixteenth aspect, the invention provides an ultrasonic diagnostic apparatus comprising the operation unit according to the fourteenth or fifteenth aspect, and a plurality of ultrasonic diagnostic apparatus main body units separate from the operation unit, wherein each of the ultrasonic diagnostic apparatus main body units includes a main body wireless communication device for wireless communicating with the operation unit, and a main body control device for accepting operation instructions issued from the operation unit via the main body wireless communication device, activating the function of the ultrasonic diagnostic apparatus in accordance with the operation instructions, and sending display data to the operation unit.

In the ultrasonic diagnostic apparatus according to the sixteenth aspect, the function of a designated ultrasonic diagnostic apparatus main body unit of a plurality of ultrasonic diagnostic apparatus main body units can be activated in accordance with operation instructions given from each operation unit. Therefore, for example, ultrasonic diagnostic apparatus main body units and sonographers are disposed every patient, and one doctor operates the plural ultrasonic diagnostic apparatus main body units substantially simultaneously, whereby one doctor is able to perform ultrasonic diagnoses on a plurality of patients substantially simultaneously. Further, since display data is sent out to each operation unit, the contents of operations and the results of operations can be displayed on the operation unit side.

According to an ultrasonic diagnostic apparatus main body unit, an operation unit and an ultrasonic diagnostic apparatus of the invention, one ultrasonic diagnostic apparatus main body unit can be operated by a plurality of operation units. Further, a plurality of ultrasonic diagnostic apparatus main body units can be operated by one operation unit.

An ultrasonic diagnostic apparatus main-body unit, operation units and an ultrasonic diagnostic apparatus according to the invention can be used where a doctor makes or provides living lessons to a sonographer and one doctor performs ultrasonic diagnoses on a plurality of patients substantially simultaneously.

Further objects and advantages of the invention will be apparent from the following description of the preferred embodiments of the invention as illustrated in the accompanying drawings, in which:
Fig. 1 is a configuration explanatory view showing an ultrasonic diagnostic apparatus according to a first embodiment.

Fig. 2 is a configuration explanatory view showing an ultrasonic diagnostic apparatus according to a second embodiment.

Fig. 3 is a configuration explanatory view showing an ultrasonic diagnostic apparatus according to a third embodiment.

The invention will be explained below in further detail by embodiments illustrated in the drawings. Incidentally, the invention is not limited to or by the embodiments.

### <First embodiment>

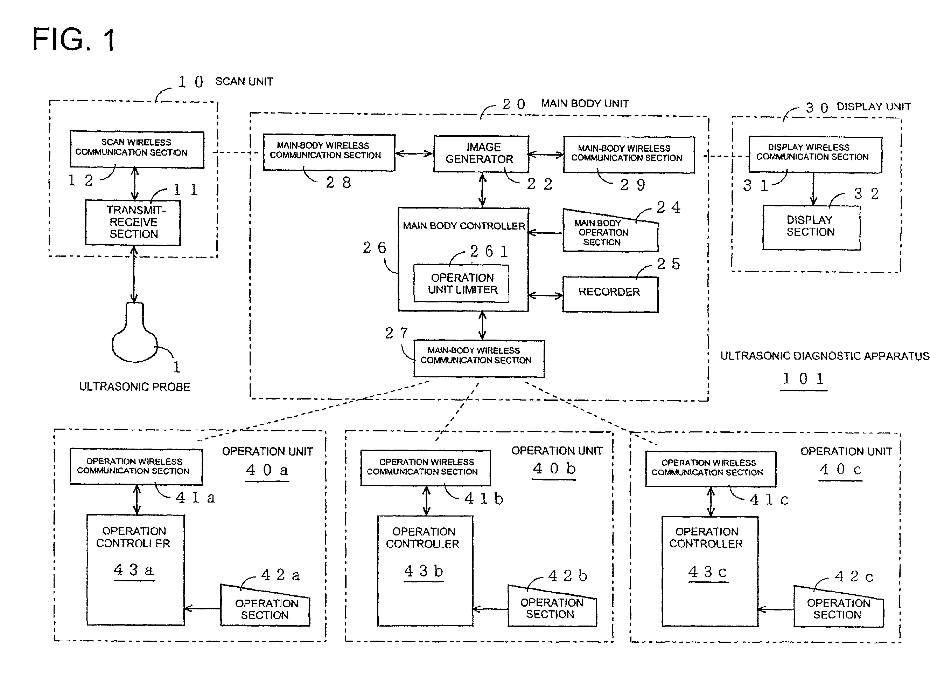

Fig. 1 is a configuration explanatory view of an ultrasonic diagnostic apparatus 101 according to a first embodiment.

The ultrasonic diagnostic apparatus 101 comprises an ultrasonic probe 1, a scan unit 10, a main body unit 20, a display unit 30 and operation units 40a, 40b and 40c that are respectively discrete bodies.

The scan unit 10 is equipped with a transmit-receive section 11 which drives the ultrasonic probe 1 to scan within a subject with an ultrasonic beam, and a scan wireless communication section 12 which wireless communicates with the main body unit 20.

The main body unit 20 is equipped with a main-body wireless communication section 28 which wireless communicates with the scan unit 10, an image generator 22 which generates an ultrasonic image, based on data obtained by scanning with the ultrasonic beam, a main body operation section 24 for supplying instructions and data by an operator, a recorder 25 which records the ultrasonic image and the like, a main body controller 26 which controls the entirety of the main body unit 20, a main-body wireless communication section 27 which wireless communicates with the operation units 40a, 40b and 40c, and a main-body wireless communication section 29 which wireless communicates with the display unit 30.

The main body controller 26 includes an operation unit limiter 261 that limits each operation unit which accepts operation instructions and releases its limitation.

The display unit 30 is equipped with a display wireless communication section 31 which wireless communicates with the main body unit 20, and a display section 32 which displays the ultrasonic image and the like thereon.

The operation unit 40a is equipped with an operation wireless communication section 41a which wireless communicates with the main body unit 20, an operation section 42a for supplying instructions and data by the operator, and an operation controller 43a which controls the entirety of the operation unit 40a.

The operation unit 40b is equipped with an operation wireless communication section 41b which wireless communicates with the main body unit 20, an operation section 42b for supplying instructions and data by the operator, and an operation controller 43b which controls the entirety of the operation unit 40b.

The operation unit 40c is equipped with an operation wireless communication section 41c which wireless communicates with the main body unit 20, an operation section 42c for supplying instructions and data by the operator, and an operation controller 43c which controls the entirety of the operation unit 40c.

The present ultrasonic diagnostic apparatus 101 is operated as follows:
(1) When the scan unit 10, the main body unit 20, the display unit 30 and the operation units 40a, 40b and 40c are powered on, mutual communications are established.
(2) The operation unit limiter 261 does not limit each operation unit that accepts operation instructions, in its initial state. All of the operations units 40a, 40b and 40c, which could establish their communications, are displayed on the display section 32.
(3) A sonographer or a doctor that operates the respective operation units 40a, 40b and 40c is capable of activating the function of the ultrasonic diagnostic apparatus by operating the operation units 40a, 40b and 40c while seeing the display section 32.
(4) Each operation unit that accepts operation instructions can be limited by operating the main body operation section 24 or the operation controller 43a, 43b or 43c. When, for example, a limitation is made so as to avoid the acceptance of operation instructions given from the operation unit 40a, only the operation units 40b and 40c are displayed on the display section 32 as active operation units.
(5) When a limitation is made due to misoperations such that the operation instructions issued from all the operation units 40a, 40b and 40c are not accepted, the main body operation section 24 is operated, thereby making it possible to restore them to the initial state.

According to the ultrasonic diagnostic apparatus 101 of the first embodiment, the function of the ultrasonic diagnostic apparatus 101 can be activated in accordance with the operation instructions issued from the plural operation units 40a, 40b and 40c. Therefore, for example, the sonographer located in the vicinity of a patient and the doctor located slightly away from the sonographer are able to operate the ultrasonic diagnostic apparatus 101 substantially simultaneously. The present embodiment is useful for allowing the doctor to make living lessons to the sonographer.

### <Second embodiment>

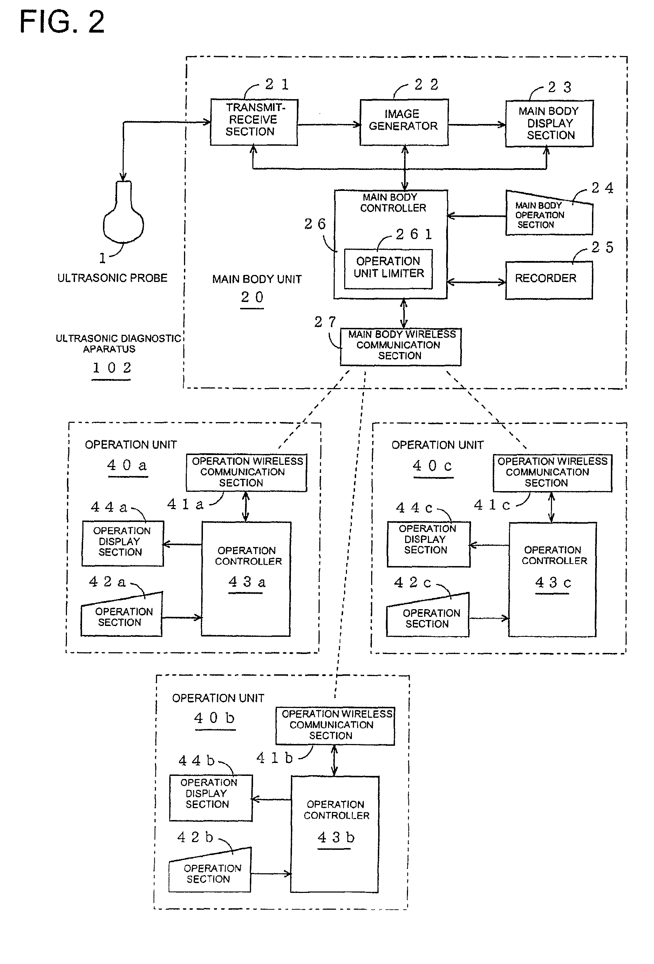

Fig. 2 is a configuration explanatory view of an ultrasonic diagnostic apparatus 102 according to a second embodiment.

The ultrasonic diagnostic apparatus 102 comprises an ultrasonic probe 1, a main body unit 20 and operation units 40a, 40b and 40c.

The scan unit 20 is equipped with a transmit-receive section 21 which drives the ultrasonic probe 1 to scan within a subject with an ultrasonic beam, an image generator 22 which generates an ultrasonic image, based on data obtained by scanning with the ultrasonic beam, a main body display section 23 which displays the ultrasonic image and the like thereon, a main body operation section 24 for supplying instructions and data by an operator, a recorder 25 which records the ultrasonic image and the like, a main body controller 26 which controls the entirety of the main body unit 20, and a main-body wireless communication section 27 which wireless communicates with the operation units 40a, 40b and 40c.

The main body controller 26 includes an operation unit limiter 261 that limits each operation unit which accepts operation instructions and releases its limitation.

The operation unit 40a is equipped with an operation wireless communication section 41a which wireless communicates with the main body unit 20, an operation section 42a for supplying instructions and data by the operator, an operation controller 43a which controls the entirety of the operation unit 40a, and an operation display section 44a which displays the contents of operations and the results of operations thereon.

The operation unit 40b is equipped with an operation wireless communication section 41b which wireless communicates with the main body unit 20, an operation section 42b for supplying instructions and data by the operator, an operation controller 43b which controls the entirety of the operation unit 40b, and an operation display section 44b which displays the contents of operations and the results of operations.

The operation unit 40c is equipped with an operation wireless communication section 41c which wireless communicates with the main body unit 20, an operation section 42c for supplying instructions and data by the operator, an operation controller 43c which controls the entirety of the operation unit 40c, and an operation display section 44c which displays the contents of operations and the results of operations.

The present ultrasonic diagnostic apparatus 102 is operated as follows:
(1) When the main body unit 20 and the operation units 40a, 40b and 40c are powered on, communications between the two are established.
(2) The operation unit limiter 261 does not limit each operation unit that accepts operation instructions, in its initial state. All of the operations units 40a, 40b and 40c, which could establish their communications, are displayed on the main body display section 23. The main body controller 26 sends display data to the respective operation units 40a, 40b and 40c.
(3) The operation display units 44a, 44b and 44c perform displays based on the display data sent from the main body unit 20. A sonographer or a doctor that operates the respective operation units 40a, 40b and 40c is capable of activating the function of the ultrasonic diagnostic apparatus by operating the operation units 40a, 40b and 40c while seeing the operation display sections 44a, 44b and 44c.
(4) Each operation unit that accepts operation instructions can be limited by operating the main body operation section 24 or the operation controller 43a, 43b or 43c. When, for example, a limitation is made so as to avoid the acceptance of operation instructions given from the operation unit 40a, only the operation units 40b and 40c are displayed on the main body display section 23 as active operation units. The operation display section 44a of the operation unit 40a displays its own inactive state, whereas the operation display sections 44b and 44c of the operation units 40b and 40c display their own active states respectively.
(5) When a limitation is made due to misoperations such that the operation instructions issued from all the operation units 40a, 40b and 40c are not accepted, the main body operation section 24 is operated, thereby making it possible to restore them to the initial state.

According to the ultrasonic diagnostic apparatus 102 of the second embodiment, the function of the ultrasonic diagnostic apparatus 102 can be activated in accordance with the operation instructions issued from the plural operation units 40a, 40b and 40c. Therefore, for example, the sonographer located in the vicinity of a patient and the doctor located slightly away from the sonographer are able to operate the ultrasonic diagnostic apparatus 102 substantially simultaneously. The present embodiment is useful for allowing the doctor to make living lessons to the sonographer. The contents of operations and the results of operations can be displayed on the operation units 40a, 40b and 40c sides.

### <Third embodiment>

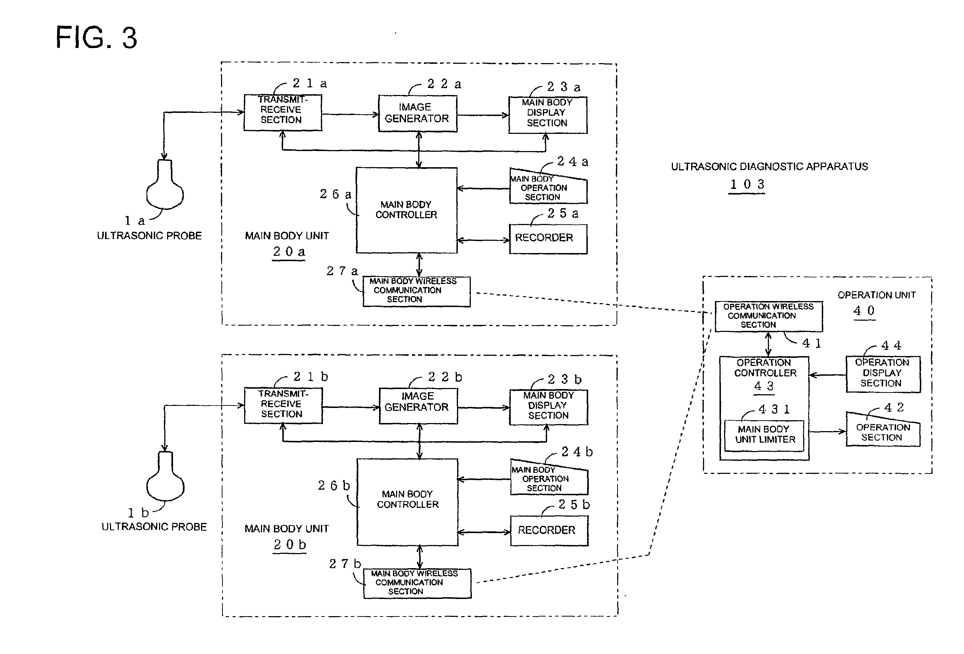

Fig. 3 is a configuration explanatory view of an ultrasonic diagnostic apparatus 103 according to a third embodiment.

The ultrasonic diagnostic apparatus 103 comprises an ultrasonic probe 1a and a main body unit 20a, an ultrasonic probe 1b and a main body unit 20b, and an operation unit 40.

The main body unit 20a is equipped with a transmit-receive section 21a which drives the ultrasonic probe 1a to scan within a subject with an ultrasonic beam, an image generator 22a which generates an ultrasonic image, based on data obtained by scanning with the ultrasonic beam, a main body display section 23a which displays the ultrasonic image and the like thereon, a main body operation section 24a for supplying instructions and data by an operator, a recorder 25a which records the ultrasonic image and the like therein, a main body controller 26a which controls the entirety of the main body unit 20a, and a main-body wireless communication section 27a which wireless communicates with the operation unit 40.

The main body unit 20b is equipped with a transmit-receive section 21b which drives the ultrasonic probe 1b to scan within a subject with an ultrasonic beam, an image generator 22b which generates an ultrasonic image, based on data obtained by scanning with the ultrasonic beam, a main body display section 23b which displays the ultrasonic image and the like thereon, a main body operation section 24b for supplying instructions and data by an operator, a recorder 25b which records the ultrasonic image and the like therein, a main body controller 26b which controls the entirety of the main body unit 20b, and a main-body wireless communication section 27b which wireless communicates with the operation unit 40.

The operation unit 40 is equipped with an operation wireless communication section 41 which wireless communicates with the main body units 20a and 20b, an operation section 42 for supplying instructions and data by the operator, an operation controller 43 which controls the entirety of the operation unit 40, and an operation display section 44 which displays the contents of operations and the results of operations thereon.

The operation controller 43 includes a main body unit limiter 431 that limits each main body unit destined for acceptance of operation instructions and releases its limitation.

The present ultrasonic diagnostic apparatus 103 is operated as follows:
(1) When the main body units 20a and 20b and the operation unit 40 are powered on, communications between the two are established.
(2) The main body unit limiter 431 sets each main body unit that could establish communications firstly as the main body unit destined for acceptance of operation instructions, as the designated main body unit in the initial state. IDs for all the main body units that could establish communications are displayed on the operation display section 44 and ID for the main body unit placed during designation at present is highlight-displayed thereon. The main body controller of the main body unit placed during designation sends display data to the operation unit 40. Further, the main body display section of the main body unit placed during designation indicates that the operation unit 40 is active, whereas the main body display section of the non-designated main body unit indicates that the operation unit 40 is inactive.
(3) The operation display unit 44 performs display based on display data sent from the main body unit placed during designation. A doctor that operates the operation unit 40 is capable of activating the function of the designated ultrasonic diagnostic apparatus by operating the operation unit 40 while seeing the operation display section 44.
(4) Operating the main body operation unit 24a or 24b or the operation section 42 makes it possible to change the main body unit destined for acceptance of the operation instructions.
(5) When all the main body units are placed out of destination for the acceptance of the operation instructions due to misoperations, the operation section 42 is operated to make it possible to restore them to the initial state.

According to the ultrasonic diagnostic apparatus 103 of the third embodiment, the function of the main body unit of the ultrasonic diagnostic apparatus, which is designated from within the plural main body units 20a and 20b, can be activated in accordance with the operation instructions issued from the operation unit 40. Therefore, for example, the main body unit of the ultrasonic diagnostic apparatus and the corresponding sonographer are placed for each patient, and one doctor operates the plural main body units 20a and 20b substantially simultaneously, whereby the single doctor is able to perform ultrasonic diagnoses on plural patients substantially simultaneously. Since the display data is sent out to the operation unit 40, the contents of operations and the results of operations can be displayed on the operation unit 40 side.

### <Fourth embodiment>

A plurality of ultrasonic diagnostic apparatus main-body units and a plurality of operation units may be associated with one another by combining the first through third embodiments.

Many widely different embodiments of the invention may be configured without departing from the spirit and the scope of the present invention. It should be understood that the present invention is not limited to the specific embodiments described in the specification, except as defined in the appended claims.

## Claims

1. An ultrasonic diagnostic apparatus main body unit (20) comprising:
a main body wireless communication device (27) for wireless communicating with separate operation units (40); and
a main body control device (26) which accepts operation instructions given from the plural operation units and activates the function of an ultrasonic diagnostic apparatus in accordance with the operation instructions.

2. The ultrasonic diagnostic apparatus main body unit (20) according to claim 1, further including an operation unit limiting device (261) for limiting each operation unit (40) that accepts the operation instructions.

3. An ultrasonic diagnostic apparatus (101) comprising:
the ultrasonic diagnostic apparatus main body unit (20) as described in claim 1 or 2; and
a plurality of operation units (40) separate from the main body unit (20),
wherein each of the operation units (40) includes an operation wireless communication device (41) for wireless communicating with the main body unit (20), an operation device (42) for inputting operation instructions to the main body unit (20) by an operator, and an operation controller (43) which sends out the operation instructions to the main body unit (20) via the operation wireless communication device (41).

4. An ultrasonic diagnostic apparatus main body unit (20) comprising:
a main body wireless communication device (27) for wireless communicating with separate operation units (40); and
a main body control device (26) which accepts operation instructions issued from the plural operation units (40) via the main body wireless communication device (27), activates the function of an ultrasonic diagnostic apparatus (102) in accordance with the operation instructions, and sends display data to the operation units (40).

5. The ultrasonic diagnostic apparatus main body unit (20) according to claim 4, further including an operation unit limiting device (261) for limiting each operation unit (40) that accepts the operation instructions.

6. An ultrasonic diagnostic apparatus (102) comprising:
the ultrasonic diagnostic apparatus main body unit (20) as described in claim 4 or 5; and
a plurality of operation units (40) separate from the main body unit (20),
wherein each of the operation units (40) includes an operation wireless communication device (41) for wireless communicating with the main body unit (20), an operation device (42) for inputting operation instructions to the main body unit (20) by an operator, an operation controller (43) which sends out the operation instructions to the main body unit (20) via the operation wireless communication device (41) and accepts display data sent from the main body unit (20), and a display device (44) which performs a display based on the display data.

7. An operation unit (40) comprising:
an operation wireless communication device (41) for wireless communicating with a plurality of separate ultrasonic diagnostic apparatus main body units (20);
an operation device (42) for designating one of the main body units (20) by an operator and inputting operation instructions to the corresponding main body unit (20) by the operator; and
an operation controller (43) which sends the operation instructions to the designated main body unit (20) via the operation wireless communication device (41).

8. An ultrasonic diagnostic apparatus comprising:
the operation unit as described in claim 7; and
a plurality of ultrasonic diagnostic apparatus main body units separate from the operation unit,
wherein each of the ultrasonic diagnostic apparatus main body units includes a main body wireless communication device (27) for wireless communicating with the operation unit, and a main body control device (26) for accepting operation instructions issued from the operation unit via the main body wireless communication device (27) and activating the function of the ultrasonic diagnostic apparatus in accordance with the operation instructions.

9. An operation unit (40) comprising:
an operation wireless communication device (41) for wireless communicating with a plurality of separate ultrasonic diagnostic apparatus main body units (20);
an operation device (42) for designating one of the main body units (20) by an operator and inputting operation instructions to the corresponding main body unit (20) by the operator;
an operation controller (43) which sends the operation instructions to the designated main body unit (20) via the operation wireless communication device (41) and accepts display data sent from the designated main body unit (20); and
a display device (44) which performs a display based on the display data.

10. An ultrasonic diagnostic apparatus (103) comprising:
the operation unit (40) as described in claim 9; and
a plurality of ultrasonic diagnostic apparatus main body units (20) separate from the operation unit (40),
wherein each of the ultrasonic diagnostic apparatus main body units (20) includes a main body wireless communication device (27) for wireless communicating with the operation unit (40), and a main body control device (26) for accepting operation instructions issued from the operation unit (40) via the main body wireless communication device (27), activating the function of the ultrasonic diagnostic apparatus (103) in accordance with the operation instructions, and sending display data to the operation unit (40).
